# EUROPEAN PATENT SPECIFICATION

(11) **EP 3 982 999 B1**
(45) Date of publication and mention of the grant of the patent: **28.01.2026**
(21) Application number: 20826394.7
(22) Date of filing: 28.05.2020
(51) Int. Cl.: A61K 38/17, A61K 48/00, A61P 9/00, A61P 11/00, A61P 13/12, C07K 14/47, A61K 9/16, A61K 33/30

(54) **COMPOSITION FOR AND METHOD OF TREATING HEPATIC TISSUE INJURY**
ZUSAMMENSETZUNG UND VERFAHREN ZUR BEHANDLUNG VON LEBERGEWEBESCHÄDEN
COMPOSITION ET MÉTHODE DE TRAITEMENT D'UNE LÉSION TISSULAIRE HÉPATIQUE

(30) Priority: 17.06.2019 US 201962862445 P
(43) Date of publication of application: 20.04.2022
(73) Proprietor: Trim-Edicine, Inc., Columbus OH 43212 (US)
(72) Inventor: ZENG, Chunyu, Chongqing, 400042 (CN); HAN, Yu, Chongqing, 400042 (CN); MA, Jianjie, Powell, OH 43065 (US); TAN, Tao, Columbus, OH 43220 (US)
(74) Representative: Elzaburu S.L.P.
(86) International application number: PCT/US2020/034977
(87) International publication number: WO 2020/256905

(56) References cited:
- WO-A1-2016/109638
- WO-A1-2018/024110
- CA-A1- 3 031 856
- US-A1- 2010 278 934
- US-A1- 2015 110 778
- US-B2- 8 420 338
- YAO WEIFENG ET AL: "MG53 anchored by dysferlin to cell membrane reduces hepatocyte apoptosis which induced by ischaemia/reperfusion injury in vivo and in vitro", vol. 21, no. 10, 12 April 2017 (2017-04-12), RO, pages 2503 - 2513, XP093053549, ISSN: 1582-1838, Retrieved from the Internet <URL:https://api.wiley.com/onlinelibrary/tdm/v1/articles/10.1111%2Fjcmm.13171> [retrieved on 20230612], DOI: 10.1111/jcmm.13171
- HAN YU ET AL: "Membrane-delimited signaling and cytosolic action of MG53 preserve hepatocyte integrity during drug-induced liver injury", JOURNAL OF HEPATOLOGY, ELSEVIER, AMSTERDAM, NL, vol. 76, no. 3, 1 November 2021 (2021-11-01), pages 558 - 567, XP086962246, ISSN: 0168-8278, [retrieved on 20211101], DOI: 10.1016/J.JHEP.2021.10.017
- CHANDLER ET AL.: "MG 53 Promotes Corneal Wound Healing and Mitigates Fibrotic Remodeling in Rodents", COMMUNICATIONS BIOLOGY, vol. 2, no. 71, 20 February 2019 (2019-02-20), pages 1 - 10, XP055776898
- DUANN PU, LI HAICHANG, LIN PEIHUI, TAN TAO, WANG ZHEN, CHEN KEN, ZHOU XINYU, GUMPPER KRISTYN, ZHU HUA, LUDWIG THOMAS, MOHLER PETER: "MG 53-Mediated Cell Membrane Repair Protects Against Acute Kidney Injury", SCIENCE TRANSLATIONAL MEDICINE, vol. 7, no. 279, 18 March 2015 (2015-03-18), pages 1 - 25, XP055776902, DOI: 10.1126/scitranslmed.3010755

## Description

### FIELD OF THE INVENTION

The present invention concerns compositions for and methods of facilitating repair of injured tissue of the liver (hepatic tissue). It also concerns compositions for preventing injury to hepatic tissue. More particularly, the invention concerns administration of MG53 protein to promote healing of injured hepatic tissue and to prevent injury of hepatic tissue.

The invention is defined in the appended set of claims.

### BACKGROUND OF THE INVENTION

MG53 protein (also referred to as mitsugumin 53 or TRIM72) is known in the art: US 7981866, WO2008/054561, WO2009/073808, US2011/0202033, US2011/0287004, US2011/0287015, US2013/0123340, WO2011/142744, WO2012/061793, US 8420338, US 9139630, US 9458465, US 9494602, US2014/0024594, WO2012/134478, WO2012/135868, US2015/0110778, WO2013/036610, US2012/0213737, WO2016/109638. Therapeutic uses thereof are described in the art.

Document WO2018024110A1 discloses a pharmaceutical composition of a MG53 mutant, a nucleic acid for coding the MG53 mutant, and the preparation method for the MG53 mutant, and uses of the MG53 mutant in the preparation of drugs for treating heart diseases, cerebrovascular diseases in diabetes mellitus, eye complications in diabetes mellitus, neuropathies in diabetes mellitus, diabetic foot diseases, kidney diseases and diseases related to cell and/or tissue damages.

Yao Weifeng et al. in "MG53 anchored by dysferlin to cell membrane reduces hepatocyte apoptosis which induced by ischemia/reperfusion injury in vivo and in vitro" (Journal of Cellular and Molecular Medicine (2017, Vol.:21, Nr.:10, Pages:2503-2513), https://doi.org/10.1111/jcmm.13171),

Han Yu et al. in "Membrane-delimited signaling and cytosolic action of MG53 preserve hepatocyte integrity during drug-induced liver injury" (Journal of Hepatology (2021, Vol.:76, Nr.:3, Pages:558-567), https://dx.doi.org/10.1016/j.jhep.2021.10.017), discloses that muscle-liver cross talk, with MG53 as a messenger, serves an important role in liver cell protection.

MG53 is present in serum derived from the blood of mice, rats, and humans (Zhu H, et al., "Amelioration of ischemia-reperfusion-induced muscle injury by the recombinant human MG53 protein" in Muscle & nerve (2015), 52, 852-858; and Liu J, et al., "Cardioprotection of recombinant human MG53 protein in a porcine model of ischemia and reperfusion injury" in Journal of molecular and cellular cardiology (2015), 80, 10-19. MG53 is predominantly expressed in skeletal and cardiac muscle and is absent from hepatocytes.

The liver, hepatic tissue, can be injured acutely or chronically. Examples of acute injury include injury caused by liver preservation, liver perfusion, stroke, hypoxia, liver transplantation, ischemia, open heart surgery, blood clot, loss of blood circulation to the liver, metabolic disease, cancer, shock, autoimmune diseases caused acute liver failure, drug-induced liver injury (DILI), e.g. Acetaminophen overdose, herbal supplements, toxins, some prescription medications, including antibiotics, nonsteroidal anti-inflammatory drugs, and anticonvulsants. Chronic injury is typified by injury caused by conditions, diseases or disorders of the liver, examples of which include cirrhosis of the liver, alcoholic liver disease, post-graft dysfunction, diabetes (which leads to fatty liver), hypercholesterolemia (which leads to cholestatic liver), primary sclerosing cholangitis, autoimmune hepatitis, DILI, hepatitis A, hepatitis B, hepatitis C, hepatitis D, hepatitis E, viral hepatitis, viral infection of the liver, Yellow Fever, American Plague, autoimmune hepatitis, and other such life-threatening (or end-stage) diseases.

Drug safety is a major health concern affecting the public, the pharmaceutical industry, regulatory agencies, and physicians. The decision to develop, approve, and prescribe a drug requires that the therapeutic benefits of medications outweigh their potential toxicities. The liver is a main target of drug toxicity because it metabolizes exogenous compounds into reactive intermediates that can cause hepatocyte damage and lead to progressive liver failure. DILI is the most frequently cited reason for abandoning compounds early in development or withdrawing them from the market after approval. Acetaminophen (or acetyl-para-aminophenol, APAP), the most widely used analgesic and pyretic drug, is dose-limited because of its potential to induce significant liver injury. Herbal and dietary supplements commonly used around the world often cause DILI as well. With the exceptions of n-acetylcysteine for APAP poisoning, glucocorticoids for immune-related reactions, and ursodeoxycholic acid for cholestatic liver injury, there are no effective antidotes for DILI. Drug-induced liver injury, a specific form of hepatic tissue injury, is a leading cause of acute liver failure, and treatment of acetaminophen-induced hepatotoxicity remains a challenge. DILI can occur acutely or chronically.

Healthy livers, in particular human and animal livers, does not express endogenous MG53, and normal tissue adjacent cancerous liver tissue does not contain any detectable amount of MG53. Polymerase transcriptase release factor (PTRF), a gene known to regulate caveolae membrane structure, is an obligatory factor for MG53-mediated nucleation of the membrane repair machinery. PTRF anchors MG53 to the acute injury site by binding exposed membrane cholesterol. Many studies have shown that PTRF is abundantly expressed in kidney, lung, heart, and skeletal muscle, but not in liver tissue or HepG2 cells. Thus, even if rhMG53 were to enter the hepatocytes, it cannot act in a conventional manner by recruiting intracellular vesicles to the injury sites for hepatocellular protection. Because of the lack of MG53 expression in hepatocytes and the fact that PTRF, an obligatory factor for MG53-mediated nucleation of the membrane repair response, is also missing in hepatocytes, it has expected in the art that repair of liver tissue would neither rely upon nor require the presence of MG53.

Drug-induced acute liver failure is a common clinical problem that can lead to high morbidity and mortality rates. Among these drugs, APAP is the number one cause of acute liver failure in the U.S. Discovery of interventions to treat DILI and halt its progression into liver failure will be of great value to the general public's health. In addition, the development of alternative means to preserve liver function in animal models of DILI can benefit the pharmaceutical industry by allowing a viable path to revive pre-clinical leads that were abandoned due to liver-toxicity for new clinical trials. Preservation of liver function in animal models of DILI by rhMG53 has the potential to benefit the pharmaceutical industry by allowing for a viable way to revive preclinical leads, which had unintended hepatotoxic side effects for advanced clinical evaluations. It would be an important advancement in the art to provide a composition for healing hepatic tissue injury.

### SUMMARY OF THE INVENTION

The present invention seeks to overcome some or all of the disadvantages inherent in the art. The present invention provides compositions as set out in the appended claims for facilitating repair of injured hepatic tissue and/or preventing injury to hepatic tissue. Our findings show MG53 mitigates DILI, such as APAP-induced liver injury and other chemical-induced liver toxicity. rhMG53 acts as an E3-ligase to polyubiquitinate RIPK3 for its degradation, thus reducing the cytotoxic effect of APAP-mediated activation of RIPK3/MLKL signaling. This finding is significant as it provides a means for pharmaceutical intervention of the MG53/RIPK3/MLKL signaling components in order to treat liver injury and disease. We have discovered that muscle-liver crosstalk occurs during liver injury with MG53 acting as a myokine that functions to protect the liver. Exogenous rhMG53 protein administered systemically has prophylactic and therapeutic benefits to treat DILI and liver injury caused by disease or substance abuse, e.g. alcohol abuse.

Disclosed herein is an aqueous organ preservation solution comprising MG53 and one or more other pharmaceutically acceptable excipients. The invention also provides a method of preserving an organ, the method comprising placing said organ in an organ preservation solution comprising MG53.

Disclosed herein is an aqueous organ perfusion solution comprising MG53 and one or more other pharmaceutically acceptable excipients. The invention also provides a method of perfusing an organ, the method comprising perfusing said organ with an organ perfusion solution comprising MG53.

MG53 is to be administered acutely or chronically to treat hepatic tissue injury. In some embodiments, exogenous MG53 is administered systemically. Systemic administration of MG53, in particular recombinant human MG53 (rhMG53), protects the liver from injury. MG53 released from skeletal muscle into circulation can facilitate repair of hepatic tissue injury. MG53 can translocate from the blood to injured hepatocytes, whereby it enters said hepatocytes to modulate receptor-interaction protein kinase 3 (RIPK3)-mediated MLKL (mixed lineage kinase domain-like) phosphorylation and translocation to the plasma membrane. MG53 also interacts with MLKL at the plasma membrane to reduce the pore-forming activity of MLKL thus preserving liver integrity.

When administered prophylactically, rhMG53 prevents liver injury; furthermore, rhMG53 preserves liver function when administered after injury.

A dosage form for use according to the invention of the invention can be administered one, two, three or more times per day. It can be administered daily, weekly, monthly, bimonthly, quarterly, semiannually, annually or even longer as needed. It can be administered every other day, five times per week, four times per week, three times per week, two times per week, once daily, twice daily, one to four times daily, continuously, or as frequently or infrequently as needed. The unit dose of each administration is independently selected upon each occurrence from the doses described in this specification or as determined to be therapeutically effective.

The dosage forms for use according to the invention can be administered systemically, e.g. intramuscularly, intravenously, intraperitoneally, subcutaneously, orally, or a combination of two or more thereof.

Another aspect provides a dosage form for use according to the invention that releases or provides MG53. The dosage form can be a non-biological dosage form or a biological dosage form. Suitable dosage forms release or provide MG53 to the hepatic tissue either directly or indirectly.

The dosage form can be a liquid, gel, solution, suspension, implant, explant, tablet, pill, sachet, bead(s), pellet(s), osmotic device or other pharmaceutically acceptable dosage form.

Another aspect provides a biological dosage form for use according to the invention that releases MG53 or enables expression of MG53 followed by release of MG53. A biological dosage form is one whose primary carrier or medium or content is a biological product. Suitable biological dosage forms include: a) viral vector, adenoviral vector, or retroviral vector that enters the hepatic tissue or circulatory system and causes expression and release of MG53, whereby said hepatic tissue is treated with MG53; b) autologous blood serum comprising added exogenous MG53; c) autologous blood serum comprising viral vector, adenoviral vector, or retroviral vector that causes expression of MG53 in cellular tissue; d) autologous blood serum comprising bioengineered hematopoietic stem cells that express and release MG53; or e) a combination of any two or more of the above.

It is also provided an autologous serum dosage form for use according to the invention comprising exogenously added MG53; comprising cells that express MG53; or comprising a viral vector that causes cells to express MG53.

The liver injury is caused by one or more of chemical-induced liver injury, drug-induced liver injury, cirrhosis of the liver or alcoholic liver disease, and can be acute injury or chronic injury, i.e. disease, disorder, or condition and is as defined in claim 1. Examples of acute injury include drug-induced liver injury (DILI). Chronic injury is typified by injury caused by conditions, diseases or disorders of the liver, examples of which include cirrhosis of the liver, alcoholic liver disease.

Disclosed herein, the dosage form is independently selected at each occurrence. A combination of two or more different dosage forms can be administered to the subject in need. Two or more different modes of administration can be employed.

Disclosed herein, the dosage form further comprises one or more zinc salts present in an amount sufficient to promote or enhance repair of injured hepatic tissue by MG53.

The disclosure exclude compositions comprising single unaltered natural product; however, said compositions may comprise mixtures of said unaltered natural product(s) along with other components thereby resulting in manmade compositions not present in nature. The disclosure exclude processes that employ solely unaltered natural processes; however, said processes may comprise a combination of said unaltered natural processes along with one or more other non-natural steps, thereby resulting in processes not present in nature. The disclosure may also include new therapeutic uses for natural products, new compositions comprising said natural products, and new methods employing said natural products.

Other features, advantages and embodiments of the invention will become apparent to those skilled in the art by the following description, accompanying examples and appended claims.

### BRIEF DESCRIPTION OF THE FIGURES

The following drawings are part of the present specification and are included to further demonstrate certain aspects of the invention. The invention may be better understood by reference to one or more of these drawings in combination with the detailed description of the specific embodiments presented herein.
FIG. 1 depicts before and after images of hepatic tissue before and after treatment with APAP in the presence of MG53 and the absence (control) of MG53.
FIGS. 2A and 2B depict charts of the relative concentration (as compared to concentration at 0 h) of MG53 in liver (FIG. 2A) and serum (FIG. 2B) over a 15-h period after APAP-induced injury.
FIG. 3 depicts cross-sectional immunofluorescent confocal images of immunohistochemically stained hepatocytes before and after treatment with APAP and in the presence and absence of MG53.
FIG. 4 depicts a chart of survival rate versus time for wild type (WT) mice, *mg53-*/*-* mice, and control mice when treated with MG53 after APAP-induced liver injury.
FIG. 5 depicts a chart of survival rate versus time for C57BL/6J mice when treated prophylactically with rhMG53 before administration of a known lethal dose of APAP.
FIG. 6 depicts a chart of serum AST level for wild-type mice when treated with APAP in the presence and absence of rhMG53 give prophylactically at 30 min before APAP administration.
FIG. 7A depicts a chart quantifying the observed necrotic area for the mice of FIG. 6.
FIG. 7B depicts images of the liver tissue for the mice of FIG. 6.
FIG. 8 depicts a chart of survival rate versus time for C57BL/6J mice when treated with rhMG53 the specified time points administration of a known lethal dose of APAP.
FIG. 9 depicts a chart of survival rate versus time for C57BL/6J mice when treated with rhMG53 12 hours after administration of a non-lethal dose (300 mg/kg) of APAP.
FIGS. 10A and 10B depict chart of viability for hepatocytes isolated from wild-type (FIG. 10A) and *dysferlin-*/*-* mice (FIG. 10B) after hypoxia/reoxygenation-induced (H/R-induced) liver injury.
FIG. 11 depicts propidium iodide stained images of injured hepatic tissue (HepG2 cells) treated with APAP in the presence and absence of MG53.
FIGS. 12A and 12B depict chart quantifying the observed necrotic area for c57BL/6J mice treated with CCl₄ or TAA and subsequently with rhMG53.
FIG. 13 depicts a chart of AST expression for the mice of FIGS. 12A and 12B.
FIGS. 14A and 14B depict charts of MG53 dose-dependent viability of APAP-injured HepG2 hepatocytes and mouse hepatocytes.

### DETAILED DESCRIPTION OF THE INVENTION

Unless specified otherwise, all embodiments of the invention comprising or employing "MG53" include all known forms of MG53.

As used herein and unless otherwise specified, the term MG53 protein refers to the MG53 protein present as the native form, optimized form thereof, mutant thereof, derivative thereof or a combination of any two or more of said forms. Native MG53 contains 477 amino acids that are well conserved in different animal species. Methods of preparing and/or isolating MG53 are known: US 7981866, WO2008/054561, WO2009/073808, US2011/0202033, US2011/0287004, US2011/0287015, US2013/0123340, WO2011/142744, WO2012/061793, US 8420338, US 9139630, US 9458465, US 9494602, US2014/0024594, WO2012/134478, WO2012/135868, US2015/0110778, WO2013/036610, US2012/0213737, WO2016/109638.

The sequence listing information for native MG53, and variants or various forms thereof, is disclosed in US7981866 and US9139630. The sequence listing information for a cDNA that encodes optimized native human MG53, or a fragment thereof, is disclosed in US9139630,

As used herein in reference to MG53, the term "mutant" means a recombinant form of MG53 having an amino acid change (replacement) of one, two, three or more amino acids in the amino acid sequence of native MG53. Mutant forms of MG53 and methods of preparing the same are known: US2015/0361146, EP3118317, WO2015/131728, US9139630.

As used herein the term "endogenous MG53", refers to MG53 present in a subject prior to treatment with a composition or dosage form according to the invention. As used herein, exogenous MG53 is nonendogenous MG53.

The present inventors have discovered that native MG53 protein is absent from healthy liver tissue; however, we unexpectedly found a substantial elevation of MG53 protein in livers of human patients with obstructive cholestatic liver disease. In addition, the anchoring machinery for MG53-mediated nucleation of intracellular vesicles at the injury site, e.g. PTRF, is missing from the liver; thus, muscle-derived MG53 cannot function in a conventional way of "patching" the injured hepatocyte.

We unexpectedly discovered that circulating MG53 protects hepatocyte injury through direct interaction with MLKL at the plasma membrane. We also unexpectedly found significant elevations of MG53 in organ recipient liver as compared to organ donor liver, meaning MG53 accumulation in hepatic tissue occurred after or during liver transplant. The accumulated MG53 was from the organ recipient.

We unexpectedly discovered that exogenous MG53 added to the circulatory system (blood) provides protection against hepatic tissue injury and provides therapeutic treatment of injured hepatic tissue. Addition of rhMG53 to APAP-treated hepatocytes improves membrane integrity and increases survival of the cells as evidenced by the reduced release of LDH and entry of fluorescent dyes. In addition to the extracellular actions of rhMG53, we found that rhMG53 can enter hepatocytes to harness drug-induced death signaling pathways. MG53 reduces MLKL phosphorylation and membrane translocation, thus leading to the restoration of MLKL-mediated disintegration of the hepatocellular membrane. Through biochemical and molecular studies, we identified RIPK3 as an E3-ligase substrate for MG53. The systemically administered rhMG53 enters the injured hepatocytes to target polyubiquitination of RIPK3 for proteasome-dependent protein degradation. In this way, the intracellular action of MG53 adds defensive value to hepatocellular protection by reducing stress-induced activation of the RIPK3-MLKL death signaling.

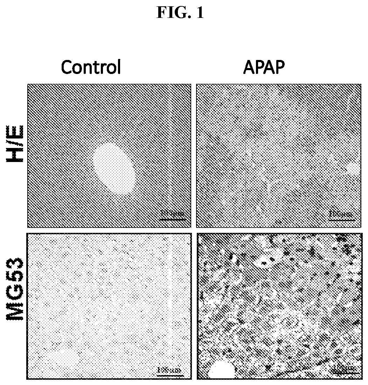

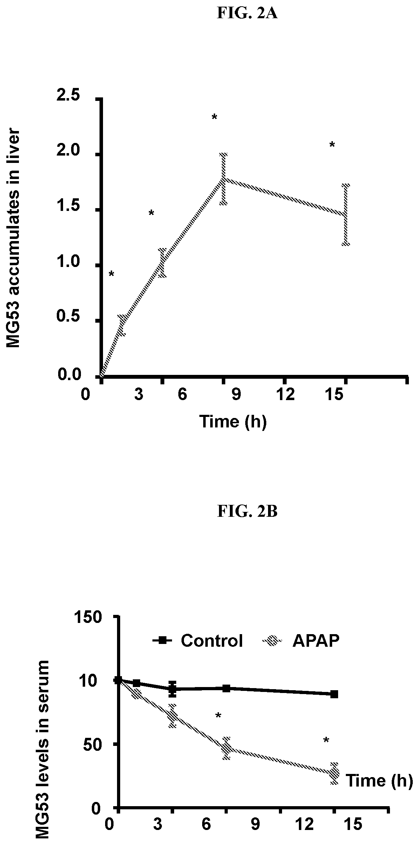

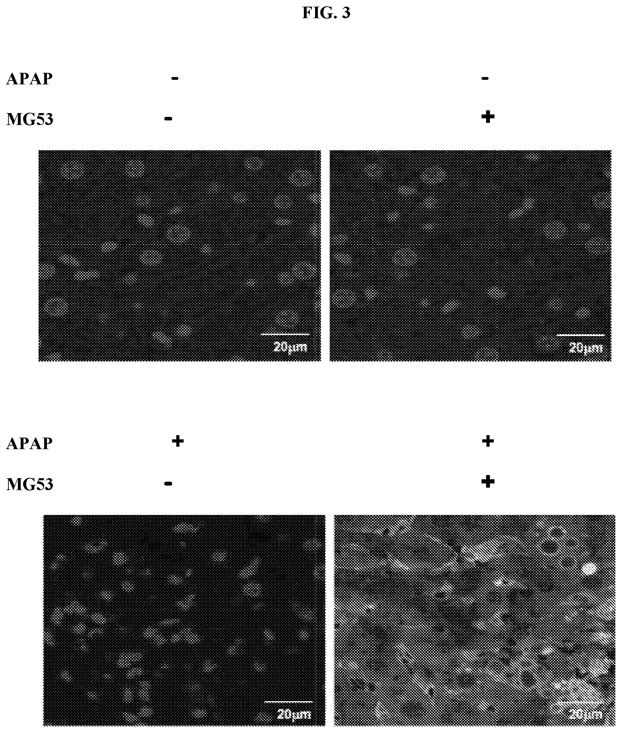

We determined that MG53 transfers from the circulatory system to the liver upon occurrence of DILI. We used a mouse model of acetaminophen-induced (APAP-induced) hepatic injury to determine whether MG53 can transfer from circulation to the liver to target the injured hepatocytes. C57BL/6J mice at the age of 8-9 weeks were treated with 350 mg APAP/kg of body weight (intraperitoneally, i.p.). At 12 hours after APAP treatment, massive liver injuries were observed based on pathological analysis. IHC staining showed a remarkable increase in MG53 accumulation in APAP-injured liver tissue (**FIG. 1**). Immunoblots with serum samples derived from the APAP-treated mice demonstrated a time-dependent decrease of MG53 protein that occurred concurrent to the increased accumulation of MG53 in the liver tissue. MG53 accumulation in the liver was evident as early as 1 hour after APAP administration (**FIG. 2A**), which mirrored the progressive decrease of MG53 proteins in circulation (**FIG. 2B**), thus indicating the transfer of MG53 from the bloodstream to injured hepatic tissue. To visualize the transfer of MG53 from the bloodstream into the injured liver, C57BL/6J mice were intravenously injected with rhMG53 protein that was conjugated with an Alexa647 fluorescent probe (Alexa647-rhMG53, 1 mg/kg) prior to APAP (350 mg/kg; i.p.) administration. As depicted in **FIG. 3**, the Alexa647-rhMG53 fluorescent signal was clearly visible in the injured hepatocytes, whereas no Alexa647-rhMG53 signal was visible in the untreated mouse liver. This finding suggests that APAP-induced liver toxicity triggers MG53 accumulation in injured hepatocytes from the bloodstream.

We determined, however, that the amount of endogenous MG53 provided by the body is on its own insufficient to overcome DILI. Pilot studies were conducted to explore the dose dependence of APAP-induced hepatotoxicity in mice. We tested four dosages of APAP (200, 350, 500, and 650 mg/kg) in wild-type mice, and found that 500 and 650 mg/kg caused lethality within 24 hours after i.p. administration. Therefore, the 350 mg/kg dose was used for the comparative studies with *mg53-*/*-* mice and their littermate wild-type controls. Histological analysis revealed the presence of typical bridging necrosis within the centrilobular region of the liver in the APAP-treated mice. Compared to wild-type littermates, a significantly higher degree of liver injuries was observed in the *mg53-*/*-* mice at 12 hours after treatment with 350 mg/kg APAP. The survival rate of APAP-treated *mg53-*/*-* mice was significantly lower than APAP-treated wild-type littermates throughout the 48-hour observation period. Aspartate aminotransferase (AST) levels in serum were significantly elevated in the *mg53-*/*-* mice during treatment with APAP, thus indicating a more severe hepatic injury compared with the wild-type mice.

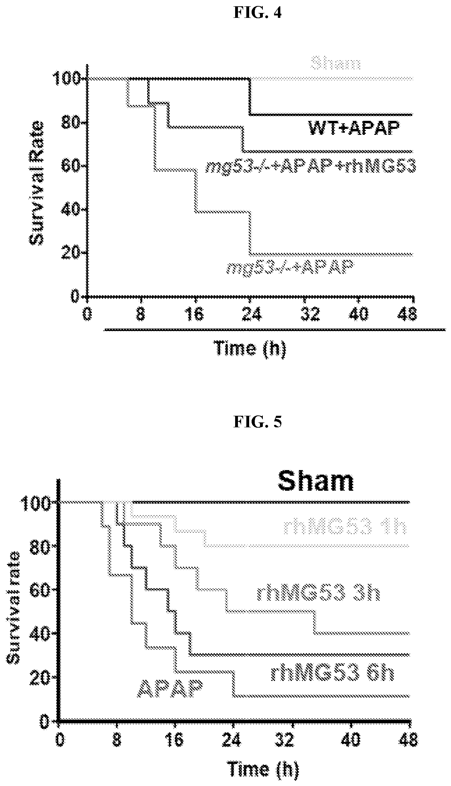

We also determined whether exogenous MG53 administered to a subject might be sufficient to prevent or reduce the severity of DILI. When exogenous rhMG53 protein was administered to the *mg53-*/*-* mice (1 mg/kg, i.p.) prior to APAP-treatment (350 mg/kg), survival of the *mg53-*/*-* mice was improved (**FIG. 4**) to a level similar to the survival curve of the wild-type mice following treatment with 350 mg/kg APAP. Moreover, *mg53-*/*-* mice receiving rhMG53 treatment also showed reduced serum levels of AST, which is consistent with improved liver integrity.

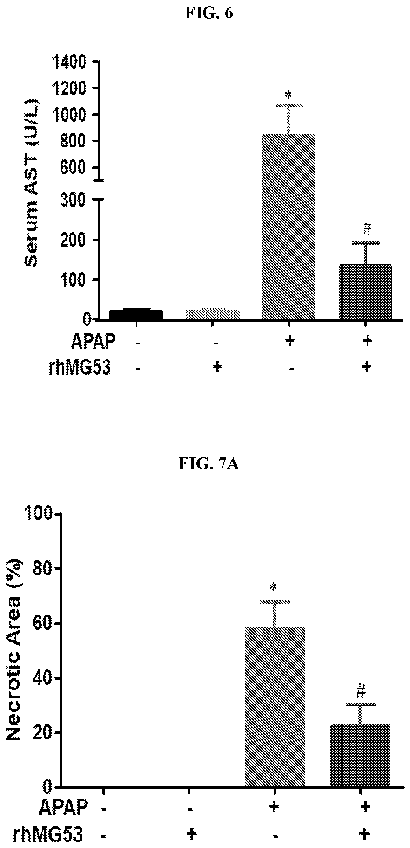

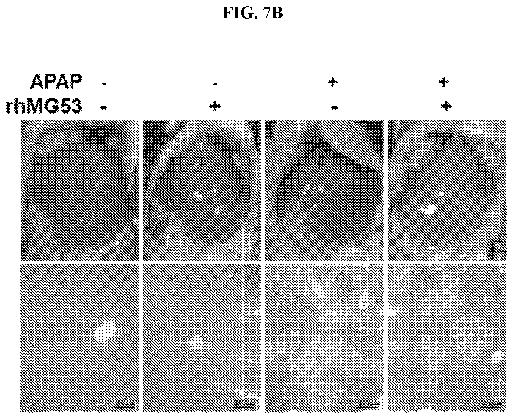

We also explored whether there was a preventive effect of rhMG53 on APAP-induced hepatic injury in C57BL/6J mice. For this purpose, rhMG53 protein (1.0 mg/kg) was intraperitoneally injected 30 minutes before the administration of 500 mg/kg APAP, a dose that caused lethality in the wild-type mice. As shown in **FIG. 5**, increased survival rate of the wild-type mice was observed with the prophylactic application of rhMG53. Moreover, pretreatment of wild-type mice with rhMG53 (1.0 mg/kg, i.p. 30 min before 350 mg/kg APAP) reduced the increased serum levels of AST (**FIG. 6**) and ameliorated necrotic injuries to the mouse liver (**FIG. 7A** and **FIG. 7B**). The exogenous rhMG53 protein was found to accumulate in the cell membrane of injured hepatic tissue collected from APAP-treated C57BL/6J mice. These data indicate that rhMG53 also has protective effects on DILI when applied prophylactically.

Not being part of the invention claimed, the disclosure provides a method of preventing or reducing the severity of hepatic tissue injury in a subject, the method comprising prophylactically (meaning before occurrence of hepatic tissue injury) administering to said subject an effective amount of exogenous MG53.

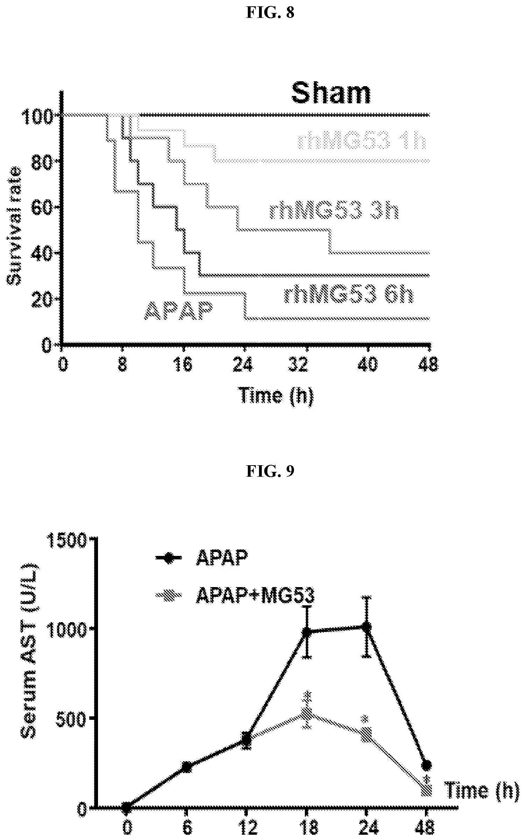

We also determined whether there was a time window within which administration of MG53 would provide a clinical or therapeutic benefit after occurrence of hepatic tissue injury. Exogenous rhMG53 (1 mg/kg, i.p.) was administered to the C57BL/6J mice at different time-points (1h, 3h, and 6h) after the administration of APAP (500 mg/kg). As determined by the survival rate, the therapeutic effect was most apparent when rhMG53 was applied at 1h after APAP; though, at even 6 hours after APAP, the therapeutic effect was still evident (**FIG. 8**).

To further determine the therapeutic window of rhMG53, a lower dose of APAP (300 mg/kg, i.p.) was used. 12 hours after APAP treatment, administration of rhMG53 (1 mg/kg) resulted in mitigation of liver injury as evidenced by the reduction of serum levels of AST (**FIG. 9**), and reduced necrotic injury to the liver tissue. These findings indicate that rhMG53 is therapeutically beneficial to injured liver after APAP-treatment.

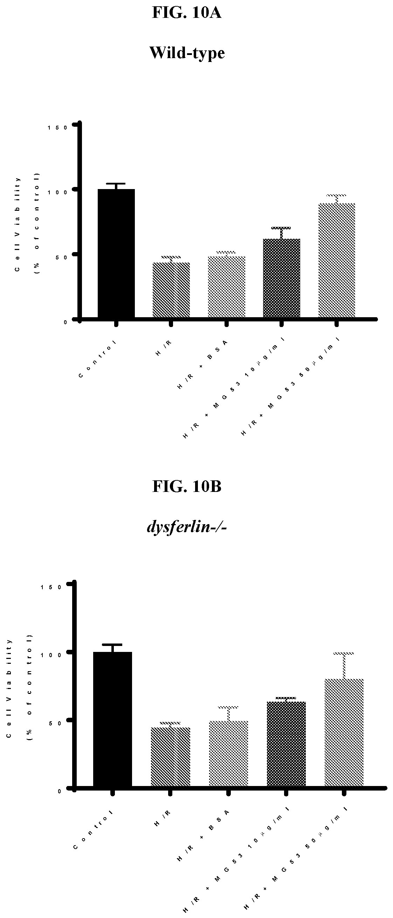

We also determined that the protective effect of MG53 could be extended beyond DILI to hypoxia/reoxygenation-induced (H/R-induced) liver injury. We isolated primary hepatocytes from wild-type and *dysferlin-*/*-* mice. Flow cytometry demonstrated >97% purity of the isolated hepatocytes from both wild-type and *dysferlin-*/*-* mice. When hepatocytes were subjected to H/R (3 hours hypoxia, 3 hours of reoxygenation), a significant reduction in cell viability was detected (**FIGS. 10A** and **10B**). With the addition of 10 µg/ml or 50 µg/ml of rhMG53, increased cell viability of hepatocytes was observed. Confocal microscopy demonstrated that exogenous rhMG53 targets the plasma membrane of injured hepatocytes as well as accumulates in the cytosol. As a control, the addition of bovine serum albumin (BSA) had no effect. We also observed a dose-dependent effect of rhMG53 in the reduction of AST in the culture medium derived from H/R-treated wild type hepatocytes.

Not being part of the invention claimed, the disclosure provides a method of treating or reducing the severity of hepatic tissue injury in a subject, the method comprising administering to said subject an effective amount of exogenous MG53, said administration being concurrently with or after at least initial occurrence of hepatic tissue injury. Said administration can be within minutes, hours, days, weeks, or months of said injury.

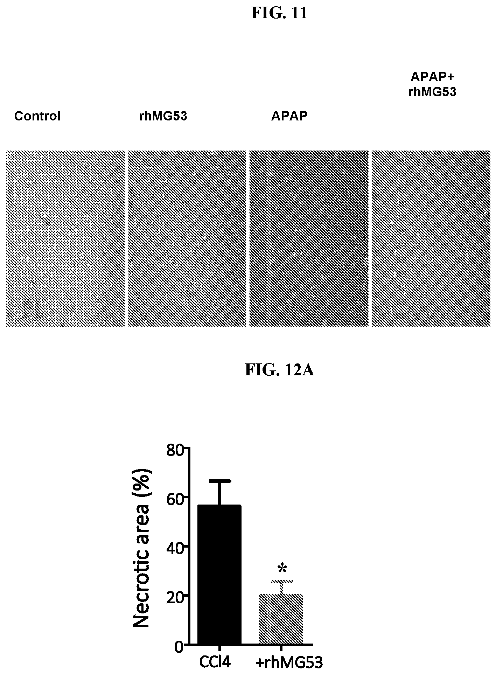

We also determined that exogenous MG53 can reduce the extent of hepatic cell death following injury of hepatic tissue. We used propidium iodide (PI) staining to quantify the necrotic cell death in HepG2 following APAP treatment. HepG2 cells treated with rhMG53 and APAP exhibited significant reduction in cell death as compared to HepG2 cells treated only with APAP (**FIG. 11**).

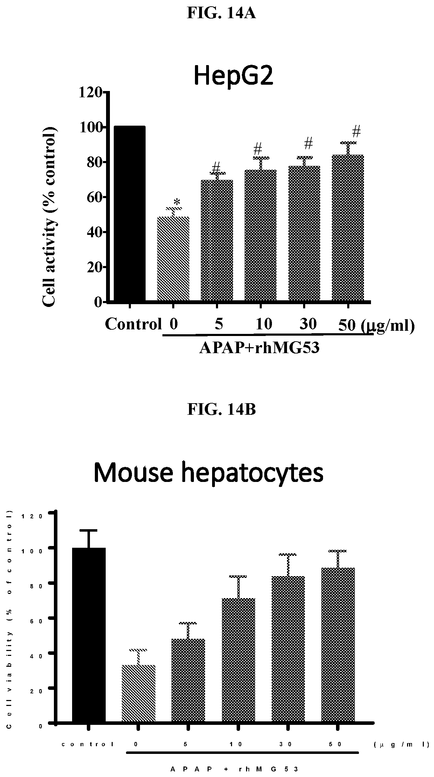

We examined the dose-dependent protective activity of MG53 against APAP-induced liver injury to HEPG2 hepatocytes and to mouse hepatocytes. The data (FIG. 14A and 14B) indicate that exogenous MG53 provides dose-dependent protective activity against APAP-induced liver injury. The data herein can be extrapolated to develop suitable protective doses for humans and large animals.

Accordingly, the disclosure provides a method of reducing the extent of cell death in injured hepatic tissue, the method comprising administering exogenous MG53 to said injured hepatic tissue, thereby reducing the number of hepatic cells that die in said injured hepatic tissue.

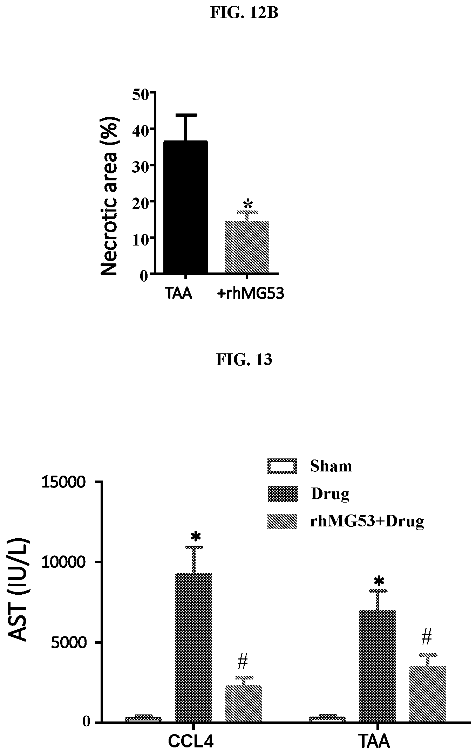

We also determined the efficacy of exogenous MG53 to protection against DILI caused by other chemicals. MG53 provided protective benefits when evaluated against carbon tetrachloride (CCl₄) and thioacetamide (TAA). C57BL/6J mice were injected with CCl₄ or TAA to cause chemical-induced liver injury and subsequently treated with MG53 via i.p. administration (Example 7). Liver tissue was biopsied and examined under microscope. The extent of necrotic tissue formation and the level of AST expression were quantified. The data in FIG. 12A, FIG. 12B, and FIG. 13 indicate exogenous rhMG53 administered after injury provides protective activity against liver tissue injury.

Accordingly, disclosed herein is a method for reducing the severity of chemical-induced liver injury, the method comprising administering to a subject having a chemical-induced liver injury an effective amount of exogenous MG53. Disclosed herein is a method of preventing or reducing the occurrence of chemical-induced liver injury, the method comprising prophylactically administering to a subject likely to be exposed to a liver injury-inducing chemical a protective or preventive amount of exogenous MG53.

The present inventors established the therapeutic efficacy of expressed MG53 in membrane repair following injury to the liver.

The present inventors established the therapeutic efficacy of exogenous rhMG53 toward healing of the liver after injury according to claim 1. The data herein indicate MG53 can be administered exogenously and prophylactically to a subject to minimize injury and improve healing of injured tissue.

MG53 expressed in vivo is effective toward healing of liver injury. In the absence of exogenously administered MG53, the injured liver tissue of *mg53-*/*-* mice exhibited increased necrosis as compared to injured liver tissue from wt mice. When exogenous MG53 was administered to the *mg53-*/*-* mice with injured liver tissue, a substantial reduction in necrosis of hepatic tissue was observed.

Further proof of the therapeutic efficacy of expressed MG53 toward treatment of liver injury was established by comparing *mg53-*/*-* mice and wt mice treated with APAP and subsequently with exogenous MG53. Immunofluorescent confocal imaging of immunohistochemical stained liver tissue was performed. Data indicate improved healing of liver tissue for both *mg53-*/*-* mice and *wt* mice when exogenous MG53 is administered before or after occurrence of chemical-induced liver injury.

The invention provides a dosage for treating liver tissue injury according to claim 1. The concentration or amount of MG53 in said dosage form is as described herein. The therapeutically effective amount of MG53 is as described herein.

It is important to observe that wt (wild-type) liver tissue is incapable of expressing normal (natural) levels of endogenous MG53. Moreover, the amount or concentration of endogenous MG53 present in the circulatory system (blood) is insufficient to provide significant clinical benefit against chronic or acute liver injury. It is by administration of exogenous MG53, by way of a dosage form comprising MG53 or causing expression of MG53 or releasing MG53, that hepatic tissue injury can be prevented, reduced, or eliminated. It is also by administration of MG53, by way of a bioengineered dosage form comprising MG53 or expressing MG53, that hepatic tissue injury can be prevented, reduced, or eliminated.

The disclosure also provides a method of increasing the expression of MG53 in hepatic tissue, the method comprising the step of administering to a subject in need thereof a viral vector that induces expression of MG53 in therapeutically effective amounts. The methods can alternatively or additionally comprise the step of administering to the subject stem cells that express MG53. Systemic administration is particularly suitable.

Suitable concentrations of MG53 in a dosage form include at least 1 ng of MG53/ml , at least 5 ng of MG53/ml, at least 10 ng of MG53/ml, at least 25 ng of MG53/ml, at least 50 ng of MG53/ml, at least 75 ng of MG53/ml, at least 100 ng of MG53/ml, at least 250 ng of MG53/ml, at least 500 ng of MG53/ml, at least 750 ng of MG53/ml, at least 1 µg of MG53/ml, at least 5 µg of MG53/ml, at least 10 µg of MG53/ml, at least 15 µg of MG53/ml, at least 20 µg of MG53/ml, at least 25 µg of MG53/ml, at least 30 µg of MG53/ml, at least 50 µg of MG53/ml, or at least 100 µg of MG53/ml. Higher concentrations are also acceptable, particularly in view the efficacy dose-response trend observed for MG53. These doses can be administered on a frequency as described herein or as determined to be most effective.

Suitable doses of MG53 that can be administered to a subject in one or more dosage forms include at least 1 ng of MG53, at least 5 ng of MG53, at least 10 ng of MG53, at least 25 ng of MG53, at least 50 ng of MG53, at least 75 ng of MG53, at least 100 ng of MG53, at least 250 ng of MG53, at least 500 ng of MG53, at least 750 ng of MG53, at least 1 µg of MG53, at least 5 µg of MG53, at least 10 µg of MG53, at least 15 µg of MG53, at least 20 µg of MG53, at least 25 µg of MG53, at least 30 µg of MG53, at least 50 µg of MG53, or at least 100 µg of MG53. Such doses can be on a total body weight basis or a per kg of body weight basis.

The dose of exogenous MG53 can be as low as about 1 up to about 1000 microg per kg of body weight.

The amount of therapeutic compound (MG53) incorporated in each dosage form will be at least one or more unit doses and can be selected according to known principles of pharmacy. An effective amount of therapeutic compound is specifically contemplated. By the term "effective amount", it is understood that, with respect to, for example, pharmaceuticals, a pharmaceutically (therapeutically) effective amount is contemplated. A pharmaceutically effective amount is the amount or quantity of a drug or pharmaceutically active substance which is sufficient to elicit the required or desired therapeutic response, or in other words, the amount which is sufficient to elicit an appreciable biological response when administered to a patient.

The term "unit dosage form" is used herein to mean a dosage form containing a quantity of the drug, said quantity being such that one or more predetermined units may be provided as a single therapeutic administration.

The dosage form is independently selected at each occurrence from the group consisting of liquid solution, suspension, gel, cream, ointment, slab gel, insert (implant).

The dosage form can also be an organ preservation solution or organ perfusion solution further comprising MG53 in a concentration as specified herein. Accordingly, the disclosure also provides an organ preservation or organ perfusion composition comprising a stock aqueous solution and MG53. The stock aqueous solution can be selected from the group consisting of Euro-Collins solution, Custodiol Histidine-Tryptophan-Ketoglutarate (HTK), or University of Wisconsin (UW) perfusion/organ preservation solution (VIASPAN). The invention also provides a method of preserving an organ, the method comprising storing said organ in an organ preservation solution comprising MG53. The invention also provides a method of perfusing an organ, the method comprising perfusing said organ with an organ perfusion solution comprising MG53. The organ preservation and organ perfusion solution are aqueous and can further comprise one or more pharmaceutically acceptable excipients as described herein or as described in Example 18. The organ can be liver, kidney, heart, muscle, skin, vein, artery, bone, brain, eye or other such organ.

The dosage form can also include autologous blood serum. Dosage forms comprising autologous (blood) serum can be made as described by Geerling et al. ("Autologous serum eye drops for ocular surface disorders" in British Journal of Ophthalmology (2004) 88:1467-1474; http://dx.doi.org/10.1136/bio.2004.044347) or by Fox et al. (Beneficial effect of tears made with autologous serum in patients with keratoconjunctivitis sicca in Arthritis Rheum. (1984), 28:459-461), or as described herein (Example 16). In some embodiments, exogenous MG53 is added to the dosage forms, or stem cells expressing MG53 are added to the dosage forms, or viral vectors that cause cells to express MG53 are added to the dosage forms, or embodiments of two or more such systems are employed in said dosage form(s).

Accordingly, the invention provides an autologous serum dosage form comprising exogenously added MG53. The invention also provides an autologous serum dosage form comprising cells that express MG53. The invention also provides an autologous serum dosage form comprising a viral vector that causes cells to express MG53.

Compositions and dosage forms of the invention can further comprise one or more pharmaceutically acceptable excipients. Dosage forms can comprise one or more excipients independently selected at each occurrence from the group consisting of acidic agent, alkaline agent, buffer, tonicity modifier, osmotic agent, water soluble polymer, water-swellable polymer, thickening agent, complexing agent, chelating agent, penetration enhancer. Suitable excipients include U.S.F.D.A. inactive ingredients approved for use in parenteral or oral formulations (dosage forms), such as those listed in the U.S.F.D.A.'s "Inactive Ingredients Database (available on the following website: https://www.fda.gov/Drugs/InformationOnDrugs/ucm113978.htm; Oct. 2018.

As used herein, an acidic agent is a compound or combination of compounds that comprises an acidic moiety. Exemplary acidic agents include organic acid, inorganic acid, mineral acid and a combination thereof. Exemplary acids include hydrochloric acid, hydrobromic acid, sulfuric acid, sulfonic acid, sulfamic acid, phosphoric acid, or nitric acid or others known to those of ordinary skill; and the salts prepared from organic acids such as amino acids, acetic acid, propionic acid, succinic acid, glycolic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, sulfanilic acid, 2-acetoxybenzoic acid, fumaric acid, toluenesulfonic acid, methanesulfonic acid, ethane disulfonic acid, oxalic acid, isethionic acid, others acids known to those of ordinary skill in the art, or combinations thereof.

As used herein, an alkaline agent is a compound or combination of compounds that comprises an alkaline moiety. Exemplary alkaline agents include primary amine, secondary amine, tertiary amine, quaternary amine, hydroxide, alkoxide, and a combination thereof. Exemplary alkaline agents include ammonia solution, ammonium carbonate, diethanolamine, monoethanolamine, potassium hydroxide, sodium borate, sodium carbonate, sodium bicarbonate, sodium hydroxide, triethanolamine, diethanolamine, monobasic phosphate salt, dibasic phosphate salt, organic amine base, alkaline amino acids and trolamine, others known to those of ordinary skill in the art, or combinations thereof.

Exemplary excipients (inactive ingredients as defined by the U.S.F.D.A.) that can be included in dosage forms of the invention include, by way of example and without limitation, water, benzalkonium chloride, glycerin, sodium hydroxide, hydrochloric acid, boric acid, hydroxyalkylphosphonate, sodium alginate, sodium borate, edetate disodium, propylene glycol, polysorbate 80, citrate, sodium chloride, polyvinylalcohol, povidone, copovidone, carboxymethylcellulose sodium, Dextrose, Dibasic Sodium Phosphate, Monobasic Sodium Phosphate, Potassium Chloride, Sodium Bicarbonate, Sodium Citrate, Calcium Chloride, Magnesium Chloride, stabilized oxychloro complex, Calcium Chloride Dihydrate, Erythritol, Levocarnitine, Magnesium Chloride Hexahydrate, Sodium Borate Decahydrate, Sodium Citrate Dihydrate, Sodium Lactate, Sodium Phosphate (Mono- and Dibasic-), Polyethylene Glycol 400, Hydroxypropyl Guar, Polyquaternium-1, Zinc Chloride, white petrolatum, mineral oil, hyaluronic acid, artificial tear, or combinations thereof.

One or more zinc salts can be included in a composition or dosage form of the invention. Such zinc salt(s) may also be administered to a subject receiving exogenous MG53 or expressed MG53. Pharmaceutically acceptable zinc salts include Zinc gluconate, Zinc acetate, Zinc sulfate, Zinc picolinate, Zinc orotate, Zinc citrate, and other such salts comprising a zinc cation and organic or inorganic anion(s).

It should be understood, that compounds used in the art of pharmaceutical formulations generally serve a variety of functions or purposes. Thus, if a compound named herein is mentioned only once or is used to define more than one term herein, its purpose or function should not be construed as being limited solely to that named purpose(s) or function(s).

As used herein, "pharmaceutically acceptable salts" refer to derivatives of the disclosed compounds wherein the compound is modified by making an acid or base salt thereof. Examples of pharmaceutically acceptable salts include, but are not limited to, mineral or organic acid salts of basic residues such as amines; alkali or organic salts of acidic residues such as carboxylic acids; and others known to those of ordinary skill. The pharmaceutically acceptable salts can be synthesized from the parent therapeutic compound which contains a basic or acidic moiety by conventional chemical methods. Lists of suitable salts are found in Remington's Pharmaceutical Sciences, 17th ed., Mack Publishing Company, Easton, PA, 1985, p. 1418.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

MG53 can be used in cotherapy or adjunctive therapy with one or more other active ingredients to treat liver diseases, disorders or conditions. Exemplary suitable active ingredients include, among others, U.S.F.D.A. approved drugs for parenteral or oral dosage forms. Such active ingredients include, by way of example and without limitation, the following. Even though specific diseases, disorders and conditions are listed for specific combinations, the invention includes other uses wherein said combinations are known or found to be therapeutically effective.

Other active ingredients that can be used in cotherapy or adjunctive therapy with MG53 include, by way of example and without limitation, N-acetylcysteine, Milk Thistle (Herbs/Suppl), colchicine, corticosteroids, curcumin, glycyrrhizin, interferons (for their antifibrotic properties), Liv 52, nitric oxide, resveratrol, silymarin, sulfoadenosylmethionine, thalidomide, zinc, and tocotrienols or combinations thereof.

The therapeutically acceptable dose, maximum tolerated dose (MTD), and minimally effective dose (MED) for each of said active ingredients is well known and set forth in the respective U.S.F.D.A. approved product package insert for each said active ingredients.

A composition, dosage form or formulation of the invention can include one, two or more active ingredients in combination with MG53. The dose of each said active ingredient in said composition, dosage form or formulation of the invention will be a therapeutically effective dose including and above the MED and including and below the MTD.

In some embodiments, the combination treatment of MG53 with another active ingredient provides at least additive therapeutic efficacy. In some embodiments, said combination provides synergistic therapeutic efficacy. In some embodiments, MG53 reduces the occurrence of, reduces the level of, or eliminates adverse events caused by the other active ingredient. In some embodiments, MG53 repairs injury caused by the other active ingredient.

Diseases, disorders or conditions that can be treated with the MG53-containing composition, dosage form or formulation of the invention (with or without additional active ingredient(s) as may be required or clinically indicated) include acute and/or chronic ones and are defined in claim 1. Examples of acute injury include drug-induced liver injury (DILI), Chronic injury is typified by injury caused by conditions, diseases or disorders of the liver, examples of which include cirrhosis of the liver, alcoholic liver disease.

The acceptable concentrations of said excipients are well known in the art and specific concentrations (amounts) thereof are set forth in the package insert or package label of known commercial products containing the same.

It should be understood, that compounds used in the art of pharmaceutics may serve a variety of functions or purposes. Thus, if a compound named herein is mentioned only once or is used to define more than one term herein, its purpose or function should not be construed as being limited solely to that named purpose(s) or function(s).

In the examples below, ranges are specified for the amount of each ingredient. Ranges including "0" as the lowest value indicate an optional ingredient. The lower limit ">0" indicates the respective material is present.

As used herein, the terms "about" or "approximately" are taken to mean a variation or standard deviation of ±10%, ±5%, or ±1% of a specified value. For example, about 20 mg is taken to mean 20 mg ±10%, which is equivalent to 18-22 mg.

As used herein, the term "prodrug" is taken to mean a compound that, after administration, is converted within a subject's body, e.g. by metabolism, hydrolysis, or biodegradation, into a pharmacologically active drug. The prodrug may be pharmacologically active or inactive. For example, a prodrug of MG53 (native or mutant) would be converted to the native form or mutant form, respectively, of MG53. The term "precursor" may also be used instead of the term "prodrug".

As used herein, the term "derivative" is taken to mean: a) a chemical substance that is related structurally to a first chemical substance and theoretically derivable from it; b) a compound that is formed from a similar first compound or a compound that can be imagined to arise from another first compound, if one atom of the first compound is replaced with another atom or group of atoms; c) a compound derived or obtained from a parent compound and containing essential elements of the parent compound; or d) a chemical compound that may be produced from first compound of similar structure in one or more steps. For example, a derivative may include a deuterated form, oxidized form, dehydrated, unsaturated, polymer conjugated or glycosilated form thereof or may include an ester, amide, lactone, homolog, ether, thioether, cyano, amino, alkylamino, sulfhydryl, heterocyclic, heterocyclic ring-fused, polymerized, pegylated, benzylidenyl, triazolyl, piperazinyl or deuterated form thereof.

In the examples below, ranges are specified for the amount of each ingredient. Ranges including "0" as the lowest value indicate an optional ingredient. Compositions with quantities of ingredients falling within the compositional ranges specified herein were made. Compositions of the invention comprising quantities of ingredients falling within the compositional ranges specified herein operate as intended and as claimed.

In view of the above description and the examples below, one of ordinary skill in the art will be able to practice the invention as claimed without undue experimentation. The foregoing will be better understood with reference to the following examples that detail certain procedures for the preparation and use of compositions according to the present invention. All references made to these examples are for the purposes of illustration. The following examples should not be considered exhaustive, but merely illustrative of only a few of the many embodiments contemplated by the present invention. The methods described herein can be followed to prepare and use compositions of the invention and to practice methods of the invention. Only examples directed to rhMG53 for use in treating or preventing liver injury caused by chemicals, drugs, cirrhosis alcoholic liver disease fit in the scope of the claims. The other examples are for reference.

### EXAMPLE 1

### rhMG53 protein production and quality control

The following process was used to produce native MG53 protein.

E. coli fermentation was used to obtain high quality (>97% purity) rhMG53 (recombinant human MG53) protein as described by Zhu et al. ("Polymerase transcriptase release factor (PTRF) anchors MG53 protein to cell injury site for initiation of membrane repair" in The Journal of biological chemistry (2011), 286, 12820-12824) and Weisleder et al. (Recombinant MG53 protein modulates therapeutic cell membrane repair in treatment of muscular dystrophy. Science translational medicine (2012), 4, 139ra185). The membrane protective activity of rhMG53 from each preparation was determined with established micro-glass bead injury assay as described previously (*ibid*).

### EXAMPLE 2

### Patients and Liver Sample Collection

This research has been carried out in accordance with the Declaration of Helsinki (2008) of the World Medical Association and has been approved by the Southwest Hospital Institutional Ethics Review Board (Chongqing, China). The corresponding written informed consent was obtained from all patients. Cholestatic liver samples (n=11) were surgically resected from patients with obstruction by biliary stones originating from the intrahepatic bile duct or common bile duct within several days of admission because of severe symptoms of biliary obstruction, such as jaundice. Control liver samples (n=9) were obtained from patients with liver metastases without cholestasis..

### EXAMPLE 3

### Collection of Donor and Recipient Human Livers

Human liver tissue was obtained from livers procured from deceased donors deemed acceptable for liver transplantation and matching liver transplant recipient explant livers at The Ohio State University Wexner Medical Center by the Comprehensive Transplant Center Human Tissue Biorepository. Wedge biopsies were obtained in the operation room and tissue was immediately submerged in RNALater (Thermo Fisher Scientific) or 10% neutral buffered formalin for at least 24 hours before flash freezing or embedding in paraffin, respectively. Informed research consent was obtained from all donors. All procedures to collect bio-specimens and clinical data were approved by The Ohio State University Institutional Review Board (IRB) under the Total Transplant Care Protocol (IRB #2017H0309) and associated Honest Broker Protocol (IRB#2017H0310). The secondary research use of the bio-specimens and data for these studies was approved by the Ohio State University IRB under a separate Liver Graft Dysfunction Protocol (IRB#2017H0403).

### EXAMPLE 4

### Histological Analysis

The liver tissue from mice was washed with ice-cold oxygenated saline to remove the blood and then fixed in 4% phosphate-buffered paraformaldehyde for 2 days at 4°C before processing for paraffin embedding. Liver slices were sectioned (4mm), mounted on slides, deparaffinized and rehydrated by incubated successively in xylene, 100% ethanol, 95% ethanol, 75% ethanol and PBS. Sections were stained with hematoxylin and eosin using standard procedures and scored according to a four-point scale 25 by an experienced histologist from the Department of Pathology at Daping Hospital under blinded conditions.

### EXAMPLE 5

### Experimental Animals

*mg53-*/*-* mice and their wild type littermates were generated, bred and genotyped as previously described (Cai, C., et al. MG53 nucleates assembly of cell membrane repair machinery. Nature cell biology 11, 56-64 (2009); Cai, C., et al. MG53 regulates membrane budding and exocytosis in muscle cells. The Journal of biological chemistry 284, 3314-3322 (2009); Cai, C., et al. Membrane repair defects in muscular dystrophy are linked to altered interaction between MG53, caveolin-3, and dysferlin. The Journal of biological chemistry 284, 15894-15902 (2009)). To ensure data reproducibility, mouse tail samples are retained and cataloged for secondary future validation, if necessary. The *mg53-*/*-* mouse line has been previously validated.

### EXAMPLE 6

### Liver transplantation in mice

A vascular cuff orthotopic mouse liver transplant model (Yokota et al., 2016) was performed by the Microsurgery Core at Nationwide Children's Hospital with appropriate IACUC approval. The donor mouse was anesthetized and subjected to cholecystectomy and insertion of a polyethylene stent (PE-10, Becton Dickinson) into the bile duct. The donor mouse was then heparinized, and the liver graft was perfused with 5.0 ml of HTK solution via the infrahepatic vena cava. The excised liver graft was prepared with cuffs on the infrahepatic vena cava and portal vein and then placed in HTK solution at 4°C. After removal of the recipient mouse native liver, the graft was orthotopically implanted by anastomosis of the suprahepatic vena cava and the cuffs were connected to the recipient portal vein and infrahepatic inferior vena cava. The abdominal wall was then closed in two layers, and the mouse was allowed to recover. Buprenorphine was administered at the start of surgery and at proper intervals to ensure minimization of pain or distress. At 24 hours post-transplant, the recipient mouse was humanely euthanized by exsanguination under anesthesia and blood samples and liver tissue samples were procured.

### EXAMPLE 7

### APAP-induced Hepatotoxicity

APAP, dimethyl sulfoxide (DMSO), and 3-(4,5-dimethylthiazol-2-yl)-2,5-diphenyltetrazolium bromide (MTT) were purchased from Sigma Aldrich (St. Louis, MO). APAP (20 mg/ml) was heated until dissolved in 0.9% saline and was made fresh for each experiment in. APAP was dosed at 300 or 500 mg/kg and injected intraperitoneally to C57BL/6J mice of both genders (8-9 weeks age). Alanine aminotransferase (ALT), aspartate aminotransferase (AST) and lactate dehydrogenase (LDH) were quantified using a standard clinical automatic analyzer or a commercial ALT, AST, and LDH assay kit (Catachem, Bridgeport, CT). Similar to APAP, CCL4 (2ml/kg) and TAA (200 mg/kg) were injected in the same way. Unless otherwise indicated, i.p. administration of rhMG53 was applied to mice subjected various forms of DILI

rhMG53 was lyophilized and stored at 4°C in a desiccator as a dry powder. The protein was diluted in 0.9% sterile saline, filtered through a 0.2-mm filter prior to administration into mice. Wild type or *mg53-*/*-* mice were randomly divided into three groups: sham, APAP administration and APAP administration with the rhMG53 treatment group. Unlike i.p. administration of rhMG53 to evaluate its prophylactic and therapeutic effects on DILI in mice, Alexa647-rhMG53 was given via left vena jugularis externa through catheterization with polyethylene tubing (outer diameter ¼ 0.965 mm; inner diameter ¼ 0.58 mm) to allow for confocal microscopic imaging of the fluorescent-labelled rhMG53 protein in the injured liver. The sham operation and APAP administration groups were treated with the same volume of saline as control.

### EXAMPLE 8

### Cell Culture

Primary hepatocytes were isolated from adult mice. To isolate primary murine hepatocytes, anesthetized and heparinized mice were subjected to a midline laparotomy and cannulation of the portal vein followed by liver perfusion with an EGTA-chelating perfusion buffer (190 mg EGTA, 900 mg glucose, 10 mL of 1 M HEPES, 400 mg KCl, 305 mg Na₂HPO₄

· 12H₂O, 8 g NaCl, 88 mg Na₂HPO₄ ·2H₂O, and 350 mg NaHCO₃ into a final volume of 1 L of sterile dH₂O). After perfusion with 0.4% collagenase buffer (560 mg CaCl₂··2H₂O, 10 mL of 1 M HEPES, 400 mg KCl, 305 mg Na₂HPO₄·12H₂O, 8g NaCl, 88 mg Na₂HPO₄·2H₂O, 350 mg NaHCO₃, and 400 mg collagenase IV into a final volume of 1 Lof sterile dH₂O), livers were minced, and cells were dispersed in culture medium; hepatocytes and nonparenchymal cells were separated using low-speed centrifugation and 40% Percoll density gradient centrifugation.

Isolated mouse hepatocytes (2 ×10⁵ /well) were cultured on 24-well collagen-coated plates in William's Medium E (Gibco, Life Technologies, Foster City, CA) with 10% FBS at 37 °C with 5% CO₂ for 4 hours. To assess the purity of primary hepatocytes, cells were subjected to BD Cytofix/Cytoperm and Fc Block (BD Biosciences) according to manufacturer's instructions. Hepatocytes were then stained with primary antibody against albumin (1ug/1X10⁶ cells; Novus Biologicals) and secondary AlexaFluor 594 antibody (1ug/1X10⁶ cells; Cellsignal) and analyzed by flow cytometry using a BD LSRFortessa (BD Bioscience)).

Hepatocytes were incubated in the presence or absence of APAP (10mM) or rhMG53 (50 µµg/mL). After 6 hours of culture, the cells were used for immunofluorescence assays, qPCR, and/or ELISA.

### EXAMPLE 9

### MTT Assay

A hepatoma-derived HepG2 cell line was purchased from the China Cell Line Bank (Beijing China). Cells were cultured in DMEM containing 100 U/ml penicillin, 100 U/ml streptomycin, 10% FBS, and 3 mM glutamine at 37 °C with 95% air, and 5% CO₂. HepG2 cells (1 × 10⁴ cells/well) or mouse hepatocytes were treated with different concentrations of rhMG53 (10 or 50 µg/ml) and APAP (10 mM) in the presence or absence of various inhibitors for 24 h. Then, an MTT solution (5 mg/ml) was added to the cells, followed by incubation for another 4 h. Subsequently, the MTT solution was discarded, DMSO was added to each well, and absorbance was measured.

### EXAMPLE 11

### Immunoprecipitation

Antibodies against RIPK3, MLKL, glyceraldehyde 3-phosphate dehydrogenase (GAPDH) and β-actin were purchased from Santa Cruz (??, MA) or Abcam (Cambridge, MA). Horseradish peroxidase-conjugated goat anti-mouse and goat anti-rabbit IgGs were obtained from Proteintech (Boston, MA).

HepG2 cells were incubated with vehicle, or APAP, rhMG53 (50 µg/ml) for 6 hours as described above. Cell lysates (800 µg protein/mL supernatant) were incubated with anti-MG53 or anti-MLKL, anti-RIPK3 antibody (1 µg/mL) for 1 hour, and protein-G agarose at 4°C for 12 hours. The immunoprecipitates were suspended in Laemmli buffer, boiled for 10 minutes, divided into 2 aliquots, electrophoresed, transferred, and subjected to immunoblotting with the anti-MLKL, anti-RIPK3, and anti-MG53 antibodies.

In one series of experiments, the sample immunoprecipitated with the anti-MG53, the anti-RIPK3 antibody was divided into 2 aliquots, one immunoblotted with the anti-MLKL, anti-RIPK3 antibody and the other immunoblotted with the anti-MG53 antibody. The data are shown as the ratio of MG53 immunoprecipitate/MLKL, RIPK3 immunoblot, and MG53 immunoprecipitate/MG53 immunoblot. In another series of experiments, we reversed the antibodies used for immunoprecipitation and immunoblotting. The samples were immunoprecipitated with the anti-MLKL, anti-RIPK3 antibody, and divided into 2 aliquots. One aliquot was immunoblotted with the anti-MG53 antibody, and the other was immunoblotted with the anti-MLKL and anti-RIPK3 antibody.

### EXAMPLE 12

### Immunoblotting

Crude extracts from dissected regions of the mouse liver were washed twice with ice-cold phosphate-buffered saline (PBS) and lysed in radio immunoprecipitation assay buffer (10mM tris-HCl (pH 7.2), 150mM NaCl, 1% NP-40, 0.5% SDS, and 0.5% deoxycholate), supplemented with a cocktail of protease inhibitors (Sigma) and phosphatase inhibitors (Thermo Scientific). Liver lysates (50µg) were separated by 10% SDS-PAGE and transferred onto polyvinylidene difluoride membranes (Millipore). The blots were blocked overnight with 5% nonfat dry milk in Tris-PBS with Tween 20 (TBST; 0.05% Tween 20 in 10 mmol/L of phosphate-buffered (isotonic saline)) at 4°C with constant shaking. Blots were subsequently incubated with antibodies against MG53 (1:500), MLKL (1:800), phosphor (p)-MLKL (1:800), RIPK3 (1:1000), p-RIPK3 (1:1000) and GAPDH (1:500) overnight in a cold room at 4°C. Membranes were then further incubated with infrared-labeled donkey anti-rabbit IRDye 800 (1:15000) at room temperature for 1 hour. Membranes were washed 3 times with TBS-T. Bound complexes were detected using the Odyssey Infrared Imaging System. Images were analyzed using the Odyssey Application Software to obtain the integrated intensities.

### EXAMPLE 13

### Immunofluorescence

Liver samples were fixed in 4% paraformaldehyde and embedded in Tissue Tek OCT compound (Electron Microscopy Sciences, Japan). Five micrometers of the frozen section were used for immunofluorescence. After blocked with 3% BSA (Roche, Basle, Switzerland), the sections were incubated with custom-made anti-MG53 antibody (1:100) followed by secondary antibody conjugated with Cy3 and FITC (1:100, Jackson Immuno-Research, West Grove, PA) in two experiments respectively. Mouse primary hepatocytes or HepG2 cells were fixed with 4% paraformaldehyde for 30 minutes and blocked with 3% BSA. Cells were then incubated with anti-MLKL antibody (1:100) and anti-RIPK3 antibody (1:100) followed by secondary antibody conjugated with Cy3 (1:100, Jackson Immuno-Research, West Grove, PA). Finally, nuclei were stained with DAPI.

### EXAMPLE 14

### FM1-43 Dye entry detection

HpeG2 were seeded in 35 mm glass bottom dishes at a density of 1 × 10⁴ cells/cm² and incubated over overnight. The cells were then treated with APAP 10 mM with or without rhMG53 (50µg/mL) for 12 hours with PBS as controls. After rinsing with Tyrode's solution, the cells were mixed with FM1-43 dye. FM1-43 is membrane impermeable and becomes fluorescent when it enters injured cells and binds to cellular lipids. Dye entry into the cells was monitored continuously with fluorescence confocal microscope (Eclipse Ti-U, Nikon Corporation, Tokyo, Japan) immediately after mixing with the dye. Consecutive live cell images were obtained at an interval of 4.1 sec/frame for 100 frames.

### EXAMPLE 15

### Autologous Blood Serum Dosage Form

### Preparation of Autologous Serum

Blood is collected from a subject. After about 0-48 h at 21C, the blood is centrifuged for about 5-20 min at 300-4000 g force. The supernatant serum is collected and then diluted 20-100% with BSS (balanced salt solution; about 0.9% wt NaCl in water) optionally containing chloramphenicol o other antibiotic or preservative. Exogenous rhMG53 (10-500 ng/ml; 50-250 ng/ml, or about 100 ng/ml) is added to the autologous serum. The serum can be administered one to ten times, one to five times or one to three times daily or any other dosing frequency determined to be therapeutically effective.

### EXAMPLE 16

### Oral Dosage Form of rhMG53 and EUDRAGIT S-100

rhMG53 is provided by TRIM-edicine, Inc. (Columbus, OH). EUDRAGIT S-100 (Poly(methacylic acid-co-methyl methacrylate) 1:2) is provided by EVONIK (https://healthcare.evonik.com/product/health-care/en/). The following procedure is used to prepared beads.

In a 100 mL beaker, add 35 mL water, and stir. While stirring add Eudragit S-100 powder (1.4 g), then and 12N NH₄OH (0.82 mL).

Add 2-hydroxypropyl)-β-cyclodextrin (0.24 g) to a 10 mL water (CD: 24 mg/mL).

Prepare a solution of MG53 (70 mg in ~15.5 mL PBS) at a pH 8. To this solution, add 10 mL of the CD solution and 10 mL of water for a total volume of 35.55 mL.

Mix the MG53/CD solution with the Eudragit solution while stirring.

Spray dry the resulting suspension to form the powdered dosage form containing MG53 (70 mg), EUDRAGIT (1.4 g), salts (130 mg), and CD (0.24 g) for a total solids content of 1.77 g or a MG53-loading of 40 mg/g of solid (4% loading). Spray drying conditions used: nozzle size- 0.6 mm; air speed- 0.3 m³/min; air outlet temp: 38 C; room temperature: 24 C; room humidity: 53%.

The powder can be included in a capsule, caplet, tablet or other oral dosage form.

### EXAMPLE 17

### Organ Preservation or Organ Perfusion Solution containing rhMG53

A suitable organ preservation or organ perfusion solution is provided, e.g. Euro-Collins solution, Custodiol Histidine-Tryptophan-Ketoglutarate (HTK), or University of Wisconsin (UW) perfusion/organ preservation solution (VIASPAN).

The Euro-Collins solution is a fluid composition comprising potassium phosphate (15 mmol / 1), potassium chloride (15 mmol / 1), potassium phosphate (42.5 mmol / 1), sodium bicarbonate (10 mmol / 1) and glucose (35 g /l).

The Custodiol HTK solution comprises Sodium chloride (15.0 mM), Potassium chloride (9.0 mM), Potassium hydrogen 2-Ketoglutarate (1.0 mM), Magnesium chloride · 6 H ₂ O (4.0 mM), Histidine · HCl · H ₂ O (18.0 mM), Histidine (180 mM), Tryptophan (2.0 mM), Mannitol (30 mM), Calcium chloride - 2H ₂ O (0.015 mM) in sterile Water for injection: pH 7.02-7.20 at 25°C (pH 7.4-7.45 at 4°C); Osmolality: 310 mosmol/kg.

UW solution comprises potassium lactobionate (100 mM), KH2PO4 (24 mM), MgSO4 (5 mM), raffinose (30 mM), adenosine (5 mM), glutathione (3 mM), allopurinol (1 mM), hydroxyethyl starch (50 g/L).

rhMG53 is mixed with organ preservation or organ perfusion solution to achieve the target concentration of rhMG53 as specified herein. The solution is then administered systemically to a subject in need thereof. Alternatively, the solution is used to preserve or perfuse an organ.

All data are expressed as mean ± S.D. Groups were compared by Student's t test and analysis of variance for repeated measures. A value of p<0.05 was considered significant.

All values disclosed herein may have standard technical measure error (standard deviation) of ± 10%. The term "about" or "approximately" is intended to mean ±10%, ±5%, ±2.5% or ±1% relative to a specified value, i.e. "about" 20% means 20±2%, 20±1%, 20±0.5% or 20±0.25%. The term "majority" or "major portion" is intended to mean more than half, when used in the context of two portions, or more than one-third, when used in the context of three portions. The term "minority" or "minor portion" is intended to mean less than half, when used in the context of two portions, or less than one-third, when used in the context of three portions. It should be noted that, unless otherwise specified, values herein concerning pharmacokinetic or dissolution parameters are typically representative of the mean or median values obtained.

The above is a detailed description of particular embodiments of the invention. Accordingly, the invention is not limited except as by the appended claims. All of the embodiments disclosed and claimed herein can be made and executed without undue experimentation in light of the present disclosure.

## Claims

1. A dosage form of recombinant human MG53 for use in treating or preventing liver injury, wherein said dosage form is administered to a subject in need thereof, and wherein said injury is caused by one or more of chemical-induced liver injury, drug-induced liver injury, cirrhosis of the liver or alcoholic liver disease.

2. The dosage form for use according to claim 1, wherein said MG53 is administered to injured liver tissue of said subject.

3. The dosage form for use according to claim 1 or 2, wherein said dosage form a) releases or provides said MG53; or b) enables expression of said MG53 followed by release of said MG53.

4. The dosage form for use according to claim 1 or 2, wherein said dosage form is a) a biological dosage form that releases said MG53 or enables expression of said MG53 followed by release of said MG53; b) an autologous serum dosage form comprising exogenously added said MG53; c) an autologous serum dosage form comprising cells that express said MG53; or d) an autologous serum dosage form comprising a viral vector that causes cells to express said MG53.

5. The dosage form for use according to claim 1, wherein said dosage form further comprises an effective amount of one or more other active ingredients.

6. The dosage form for use according to claim 5, wherein said dosage form is administered systemically.

7. The dosage form for use according to claim 5, wherein said biological dosage form is autologous blood serum comprising added exogenous said MG53.

8. The dosage form for use according to claim 1 or 2, wherein said dosage form is a) administered intramuscularly, intravenously, subcutaneously, orally or a combination of two or more thereof; b) administered acutely or chronically; or c) administered every other day, five times per week, four times per week, three times per week, two times per week, once daily, twice daily, one to four times daily or continuously.

9. The dosage form for use according to claim 1 or 2, wherein said injury is selected from the group consisting of an acute liver injury and chronic liver injury.

## Patentansprüche

1. Eine Dosierungsform von rekombinantem humanem MG53 zur Verwendung bei der Behandlung oder Vorbeugung einer Leberschädigung, wobei die Dosierungsform einem Subjekt verabreicht wird, das dies benötigt, und wobei die Schädigung durch eine oder mehrere von chemisch induzierter Leberschädigung, arzneimittelinduzierter Leberschädigung, Leberzirrhose oder alkoholischer Lebererkrankung verursacht wird.

2. Dosierungsform zur Verwendung nach Anspruch 1, wobei das MG53 an geschädigtes Lebergewebe des Subjekts verabreicht wird.

3. Die Dosierungsform zur Verwendung nach Anspruch 1 oder 2, wobei die Dosierungsform a) das MG53 freisetzt oder bereitstellt; oder b) die Expression des MG53 ermöglicht, gefolgt von der Freisetzung des MG53.

4. Die Dosierungsform zur Verwendung nach Anspruch 1 oder 2, wobei die Dosierungsform a) eine biologische Dosierungsform ist, die das MG53 freisetzt oder die Expression des MG53 ermöglicht, gefolgt von der Freisetzung des MG53; b) eine autologe Serumdosierungsform, die exogen zugegebenes MG53 umfasst; c) eine autologe Serumdosierungsform, die Zellen umfasst, die das MG53 exprimieren; oder d) eine autologe Serumdosierungsform, die einen viralen Vektor umfasst, der Zellen veranlasst, das MG53 zu exprimieren.

5. Dosierungsform zur Verwendung nach Anspruch 1, wobei die Dosierungsform ferner eine wirksame Menge eines oder mehrerer anderer Wirkstoffe umfasst.

6. Dosierungsform zur Verwendung nach Anspruch 5, wobei die Dosierungsform systemisch verabreicht wird.

7. Dosierungsform zur Verwendung nach Anspruch 5, wobei die biologische Dosierungsform autologes Blutserum ist, das zugesetztes exogenes MG53 umfasst.

8. Dosierungsform zur Verwendung nach Anspruch 1 oder 2, wobei die Dosierungsform a) intramuskulär, intravenös, subkutan, oral oder als eine Kombination von zwei oder mehreren davon verabreicht wird; b) akut oder chronisch verabreicht wird; oder c) jeden zweiten Tag, fünfmal pro Woche, viermal pro Woche, dreimal pro Woche, zweimal pro Woche, einmal täglich, zweimal täglich, ein bis viermal täglich oder kontinuierlich verabreicht wird.

9. Dosierungsform zur Verwendung nach Anspruch 1 oder 2, wobei die Schädigung aus der Gruppe ausgewählt ist, bestehend aus einer akuten Leberschädigung und einer chronischen Leberschädigung.

## Revendications

1. Forme posologique de MG53 humaine recombinante pour une utilisation dans le traitement ou la prévention d'une lésion hépatique, dans laquelle ladite forme posologique est administrée à un sujet qui en a besoin, et dans laquelle ladite lésion est causée par une ou plusieurs lésions parmi une lésion hépatique induite par des produits chimiques, une lésion hépatique induite par des médicaments, une cirrhose du foie ou une maladie hépatique alcoolique.

2. Forme posologique pour une utilisation selon la revendication 1, dans laquelle ladite MG53 est administrée au tissu hépatique blessé dudit sujet.

3. Forme posologique pour une utilisation selon la revendication 1 ou 2, dans laquelle ladite forme posologique a) libère ou fournit ladite MG53 ; ou b) permet l'expression de ladite MG53 suivie de la libération de ladite MG53.

4. Forme posologique pour une utilisation selon la revendication 1 ou 2, dans laquelle ladite forme posologique est a) une forme posologique biologique qui libère ladite MG53 ou permet l'expression de ladite MG53 suivie de la libération de ladite MG53 ; b) une forme posologique de sérum autologue comprenant ladite MG53 ajoutée de manière exogène ; c) une forme posologique de sérum autologue comprenant des cellules qui expriment ladite MG53 ; ou d) une forme posologique de sérum autologue comprenant un vecteur viral qui amène les cellules à exprimer ladite MG53.

5. Forme posologique pour une utilisation selon la revendication 1, dans laquelle ladite forme posologique comprend en outre une quantité efficace d'un ou plusieurs autres principes actifs.

6. Forme posologique pour une utilisation selon la revendication 5, dans laquelle ladite forme posologique est administrée par voie systémique.

7. Forme posologique pour une utilisation selon la revendication 5, dans laquelle ladite forme posologique biologique est un sérum sanguin autologue comprenant ladite MG53 ajoutée de manière exogène.

8. Forme posologique pour une utilisation selon la revendication 1 ou 2, dan laquelle ladite forme posologique est a) administrée par voie intramusculaire, intraveineuse, sous-cutanée, orale ou via une combinaison d'au moins deux de ces voies d'administration ; b) administrée de manière aiguë ou chronique ; ou c) administrée tous les deux jours, cinq fois par semaine, quatre fois par semaine, trois fois par semaine, deux fois par semaine, une fois par jour, deux fois par jour, une à quatre fois par jour ou en continu.

9. Forme posologique pour une utilisation selon la revendication 1 ou 2, dans laquelle ladite lésion est choisie dans le groupe constitué d'une lésion hépatique aiguë et d'une lésion hépatique chronique.
